(19) Europäisches Patentamt
European Patent Office
Office européen des brevets

(11) EP 4 678 163 A2

(12) EUROPEAN PATENT APPLICATION
published in accordance with Art. 153(4) EPC

(43) Date of publication:
**14.01.2026 Bulletin 2026/03**

(21) Application number: **23926529.1**

(22) Date of filing: **23.11.2023**

(51) International Patent Classification (IPC):
***A61K 9/00*** *(2006.01)* ***A61K 47/10*** *(2017.01)*
***A61K 47/14*** *(2017.01)* ***A61K 47/20*** *(2006.01)*
***A61K 9/06*** *(2006.01)* ***A61K 31/4178*** *(2006.01)*
***A61K 31/454*** *(2006.01)* ***A61K 31/69*** *(2006.01)*
***A61P 31/10*** *(2006.01)*

(52) Cooperative Patent Classification (CPC):
**A61K 9/00; A61K 9/06; A61K 9/08; A61K 31/4178; A61K 31/454; A61K 31/69; A61K 47/10; A61K 47/14; A61K 47/18; A61K 47/20; A61K 47/22; A61P 31/10**

(86) International application number:
**PCT/KR2023/018987**

(87) International publication number:
**WO 2024/185956 (12.09.2024 Gazette 2024/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **03.03.2023 KR 20230028512**

(71) Applicant: **Chang, Byeung Mo**
**Seoul 04202 (KR)**

(72) Inventor: **Chang, Byeung Mo**
**Seoul 04202 (KR)**

(74) Representative: **Cabinet Chaillot**
**16/20, avenue de l'Agent Sarre**
**B.P. 74**
**92703 Colombes Cedex (FR)**

# (54) PHARMACEUTICAL COMPOSITION FOR PREVENTING OR TREATING NAIL AND SKIN TINEA

(57) Disclosed is a pharmaceutical composition for preventing or treating nail and skin tinea. The pharmaceutical composition for preventing or treating nail and skin tinea according to one embodiment may comprise: an antifungal agent; a crystal growth inhibitor; and a keratin affinity inhibitor. The composition according to one embodiment is a pharmaceutical composition which, as a topical preparation containing the antifungal agent, inhibits crystal growth during storage, prevents crystal precipitation after application, inhibits keratin affinity, and thereby has improved skin and nail permeability and antimicrobial power. The composition may comprise the antifungal agent as an active ingredient, and the crystal growth inhibitor, the keratin affinity inhibitor, a chelating agent, and additives (e.g., a vehicle, an antioxidant, or a pH adjuster) as auxiliary ingredients.

【Figure 1】

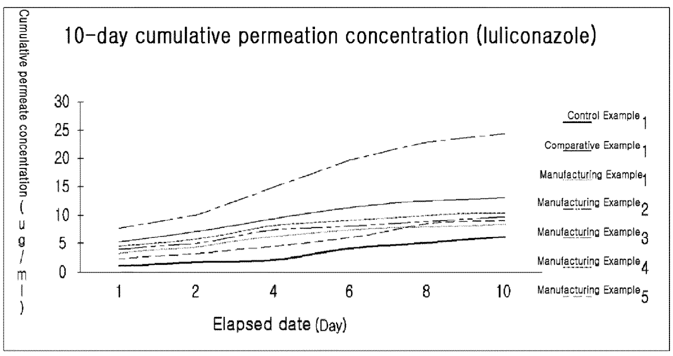


EP 4 678 163 A2

## Description

[Technical Field]

The present invention relates to a pharmaceutical composition for preventing or treating nail and skin tinea.

[Background Art]

Tinea is a general term for superficial mycoses caused by dermatophytes. Dermatophytes are keratinophilic fungi that parasitize the keratin of the stratum corneum of the epidermis, hair, fingernails, and toenails, and have keratinase that can dissolve keratin.

Typically, tinea is classified into head tinea (Tinea capitis), body tinea (Tinea coporis), jock itch (Tinea cruris), Tinea barbae, face tinea (Tinea faciei), hand tinea (Tinea manus), foot tinea (Tinea pedis), and nail tinea (Tinea unguium) depending on the site of occurrence [Byung Ho Oh, Kyu Joong Ahan. "Drug Therapy of Dermatophytosis" J Korean Med. Assoc. 2009, 52(11):1109-1114].

The causative organism for tinea may vary depending on the site of occurrence and the patient's age, but common causative organisms include Trichophyton rubrum, Trichophyton mentagrophytes, Epidermophyt floccosum, Microsporum canis, Candida albicans as a yeast, and Aspergillus niger as a fungus.

Among the various causative organisms of tinea, Trichophyton rubrum accounts for 60% and Trichophyton mentagrophytes accounts for 20%. Nail tinea is particularly common in people over 40 years of age, and Trichophyton rubrum accounts for 91.0% and Trichophyton mentagrophytes accounts for 7.7% of nail tinea causative organisms [Uwe Wollina, Holger Haenssle, Dtsch Arztebl Int. 2016;113(29-30):509-518].

Nail tinea can be classified into ① distal and lateral subungual onychomycosis (DLSO), ② proximal subungual onychomycosis (PSO), ③ white superficial onychomycosis (WSO), ④ endonyx onychomycosis (EO), and ⑤ total dystrophic onychomycosis (TDO) depending on the route and location of fungal invasion into the nail.

Meanwhile, antifungal agents are broadly classified into polyenes, azoles, allylamines, benzylamines, morpholines, hydroxypyridones, and oxaboroles. Among these, luriconazole (1% cream/5% solution), efinaconazole (10% solution), terbinafine (1% cream/spray), amorolfine (5% nail lacquer), ciclopirox (8% nail lacquer), and tavaborole (5% solution) are widely sold as treatments for onychomycosis.

However, most antifungal agents have low solubility, so there is a problem that crystals are easily formed due to temperature changes during storage, or when the drug is applied to the affected area and dried. This pattern of crystal precipitation can be explained by the Ostwald ripening theory, which states that particles with a high equilibrium concentration and small particle size in a solution dissolve into particles with a low equilibrium concentration and large particle size, forming larger particles.

In addition, existing antifungal agents have encountered technical limitations in that their preventive and therapeutic effects on tinea are reduced due to the significant decrease in drug penetration effect caused by their high affinity for keratin in the skin and nails.

Meanwhile, currently widely sold antifungal products include Jubulia (Efinaconazole 10%), which contains cyclomethicone and ethylenediamine tetraacetic acid salt, Luconac (Luliconazole 5%), which contains N-methylpyrrolidone, and Kerydin (Tavabole 5%), which contains ethylenediamine tetraacetic acid salt.

However, cyclomethicone's use is restricted in each country due to its environmental hazards, and methylpyrrolidone is classified as a carcinogen, mutagen, or reproductive toxicant, and its use is strictly regulated as a hazardous substance. In addition, ethylenediamine tetraacetic acid salt, which has been used as a chelating agent for a long time, has poor biodegradability and is not desirable for use from an environmental perspective as it may deposit heavy metals in soil and water and cause heavy metals to enter the food chain.

Therefore, there is a need for the development of a new technology that can not only resolve the problems caused by harmful ingredients contained in currently commercially available products, but also improve the solubility of antifungal agents by inhibiting crystal growth, and significantly improve the skin and nail penetration ability and antimicrobial effect by reducing the keratin affinity of antifungal agents.

[Prior Art Documents]

[Non-Patent Documents]

(Non-Patent Document 1) 1. Byung Ho Oh, Kyu Joong Ahan. "Drug Therapy of Dermatophytosis" J Korean Med. Assoc. 2009, 52(11):1109-1114.

(Non-Patent Document 2) 2. Uwe Wollina, Holger Haenssle, Dtsch Arztebl Int. 2016; 113(29-30):509-518.

[Disclosure]

[Technical Problem]

The technical idea of the present disclosure is to solve the above-described problems, and the object thereof is to provide a technology that can improve storage stability and enhance the therapeutic effect of a drug by preventing an antifungal agent from precipitating into crystals.

In addition, the technical idea of the present disclosure has another object of providing a technology for increasing the penetration ability of the skin and nail by reducing the keratin affinity of an antifungal agent.

In addition, another purpose of the technical idea of the present disclosure is to provide a technology that can fundamentally solve problems arising from the use of cyclomethicone, methylpyrrolidone, and ethylenediamine tetraacetic acid salt.

The problems to be solved by the present invention are not limited to the problems described above, and other technical problems not mentioned will be clearly understood by those skilled in the art to which the present invention pertains from the contents described below.

[Technical Solution]

In order to achieve these objects, as one embodiment of the present invention, a pharmaceutical composition for preventing or treating nail and skin tinea includes an antifungal agent; a crystal growth inhibitor; a keratin affinity inhibitor; and an additive, wherein the antifungal agent comprises efinaconazole, and the content of the antifungal agent is 10% by weight based on the total weight of the composition, the crystal growth inhibitor comprises 1,2-pentanediol and acetyltributyl citrate in a weight ratio of 1:2, and the content of the crystal growth inhibitor is 0.1 to 30% by weight based on the total weight of the composition, the keratin affinity inhibitor comprises cystamine dihydrochloride or octenidine dihydrochloride, and the content of the keratin affinity inhibitor is 0.01 to 10% by weight based on the total weight of the composition, and the causative organism of the tinea comprises at least one selected from Trichophyton rubrum, Trichophyton mentagrophytes, and Candida albicans.

Meanwhile, the pharmaceutical composition for the prevention or treatment of tinea may further include a chelating agent.

And, the content of the chelating agent can be applied at 0.0001 to 5 wt% based on the weight of the composition.

Additionally, the chelating agent may include trisodium ethylenediamine disuccinate.

Furthermore, the additive may include at least one selected from a vehicle, an antioxidant, and a pH adjuster.

And, the pharmaceutical composition for the prevention or treatment of tinea can be prepared as at least one formulation selected from a liquid, an aerosol, a stick, and a semi-solid preparation.

Additionally, the semi-solid preparation may include at least one selected from a gel, a lotion, a cream, and an ointment.

Additionally, the pharmaceutical composition for the prevention or treatment of tinea may have a pH of 2 to 10.

Additionally, the pharmaceutical composition for the prevention or treatment of tinea may have a density of 0.5 to 2 $g/cm^3$.

Additionally, the pharmaceutical composition for the prevention or treatment of tinea may have a moisture content of 0.1 to 90 wt%, as measured by the Karl Fischer method.

Additionally, the surface tension of the pharmaceutical composition for the prevention or treatment of tinea may be 1 to 76 dyn/cm.

The above-described solutions to the problems are merely exemplary and should not be construed as limiting the present invention. In addition to the exemplary embodiments described above, additional embodiments may exist as described in the drawings and detailed description of the invention.

[Advantageous Effects]

As described above, according to various embodiments of the present invention, the solubility of drugs can be improved by inhibiting crystal growth of luliconazole, efinaconazole, and tavaborole, which are poorly soluble antimicrobial agents as active ingredients. Therefore, the phenomenon of crystal precipitation does not occur even when the temperature changes during long-term storage, and when the drug is applied to the affected area of the skin or nail, the formation of fine precipitates in the affected area after the volatile solvent evaporates is prevented, thereby significantly improving the drug permeability.

In addition, according to various embodiments of the present invention, the crystal growth of the drug is

suppressed, so that no precipitate is formed, and the antimicrobial activity is excellent.

In addition, according to various embodiments of the present invention, when applying the drug to the affected area of the skin and nail, the keratin affinity of the antimicrobial agent, which is an active ingredient, is suppressed, thereby further increasing the drug permeability to have an excellent effect in preventing and treating skin tinea and nail tinea.

In addition, according to various embodiments of the present invention, the effect of preventing crystal precipitation after drug application is excellent, so that medication compliance is high, and the drug permeability and antimicrobial power in the skin and nails are very excellent.

In addition, it does not contain cyclomethicone and N-methylpyrrolidone, which are regulated substances contained in products currently on the market, and ethylenediamine tetraacetic acid salt, which has poor biodegradability, so it is less harmful and more environmentally friendly than products containing these ingredients, and can be widely used as a pharmaceutical composition for topical preparations for the prevention or treatment of skin tinea and nail tinea.

The effects according to various embodiments of the present invention are not limited to the effects mentioned above, and other effects not mentioned will be clearly understood by those skilled in the art from the description of the claims.

[Description of Drawings]

FIG. 1 is a graph showing the cumulative permeate concentration results of luriconazole for 10 days according to Control Example 1, Comparative Example 1, and Manufacturing Examples 1 to 5.

FIG. 2 is a graph showing the cumulative permeate concentration results of efinaconazole for 10 days according to Control Example 2, Comparative Example 2, and Manufacturing Examples 6 to 10.

FIG. 3 is a graph showing the cumulative permeate concentration results of tavaborole for 10 days according to Control Example 3, Comparative Example 3, and Manufacturing Examples 11 to 15.

[Best Mode]

A preferred embodiment of the present invention will be described in more detail with reference to the attached drawings, but technical parts already known will be omitted or compressed for the sake of brevity.

It should be noted that references in this specification to "one" or "an" embodiment of the invention are not necessarily to the same embodiment, but rather mean at least one.

In the examples below, singular expressions include plural expressions unless the context clearly indicates a different meaning.

In the examples below, the terms such as "include" or "have" mean that a feature or component described in the specification is present, and do not preclude the possibility that one or more other features or components may be added.

As used herein, the term "prevention" means any act of inhibiting or delaying the occurrence, spread or recurrence of a disease by administering a pharmaceutical composition according to various embodiments of the present invention.

As used herein, the term "treatment" means any action by which the symptoms of a disease are improved or beneficially changed by administration of a pharmaceutical composition according to various embodiments of the present invention.

As used herein, the term "administration" means providing an active ingredient to a subject by any suitable method.

The term "subject" as used herein includes, but is not limited to, mammals including humans, guinea pigs, monkeys, cows, horses, sheep, pigs, chickens, turkeys, quails, cats, dogs, mice, rats or rabbits, and preferably may be humans.

In this specification, the term "topical preparation" means a medicine administered by applying, spraying, or attaching to the skin.

As used herein, the term "pharmaceutically acceptable" means physiologically acceptable and does not typically cause allergic reactions such as gastrointestinal upset, dizziness, or similar reactions when administered to humans.

As used herein, the term "salt" means an acid addition salt formed by a pharmaceutically acceptable free acid. Accordingly, pharmaceutically acceptable salts refer to salts commonly used in the pharmaceutical industry, for example, inorganic ion salts manufactured with calcium, potassium, sodium or magnesium, etc.; inorganic acid salts manufactured with hydrochloric acid, nitric acid, phosphoric acid, hydrobromic acid, iodic acid, perchloric acid or sulfuric acid, etc.; organic acid salts manufactured with acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbic acid, carbonic acid or vanillic acid, etc.; sulfonic

acid salts manufactured with methanesulfonic acid, ethanesulfonic acid, benzenesulfonic acid, salicylic acid, p-toluenesulfonic acid or naphthalenesulfonic acid, etc.; amino acid salts manufactured with glycine, arginine or lysine, etc.; or amine salts manufactured with trimethylamine, triethylamine, ammonia, pyridine or picoline, etc. However, the types of salts referred to herein are not limited by these listed salts.

In this specification, the term "density" refers to a value indicating mass per unit volume, generally expressed in units of $g/cm^3$. The density described in this specification can be measured by a surface tension meter (Easy Dyne surface tension meter model K20) sold by KRÜSS company.

In this specification, the term "moisture content" means the content of moisture contained in a pharmaceutical composition expressed as a percentage. The moisture content described in this specification is measured by the Karl Fischer titration method, and a Metrohm moisture meter (Metrohm 901 KF Titrando) can be used for the measurement.

As used herein, the term "surface tension", generally expressed in units of dyn/cm, means the force required to increase the unit area of a liquid surface or the unit area of an interface between two liquids or between a liquid and a gas. The surface tension described in this specification is measured by the Du Noüy Ring Method using a surface tension meter (Easy Dyne surface tension meter model K20) sold by KRÜSS company.

A pharmaceutical composition for the prevention or treatment of tinea according to one embodiment may include an antifungal agent as an active ingredient and may further include auxiliary ingredients (e.g., crystal growth inhibitor, keratin affinity inhibitor, chelating agent, additive, etc.). In one specific example, the pharmaceutical composition for the prevention or treatment of skin tinea and nail tinea can be implemented as a pharmaceutical composition for topical application containing an antifungal agent.

A pharmaceutical composition for the prevention or treatment of tinea according to one embodiment may include an antifungal agent, a crystal growth inhibitor, and a keratin affinity inhibitor. In one embodiment, the content of the antifungal agent may be 0.1 to 20 wt% based on the total weight of the composition.

As a specific example, the content of the antifungal agent can be set to 0.1 wt%, 0.2 wt%, 0.3 wt%, 0.4 wt%, 0.5 wt%, 0.6 wt%, 0.7 wt%, 0.8 wt%, 0.9 wt%, 1 wt%, 1.5 wt%, 2 wt%, 2.5 wt%, 3 wt%, 3.5 wt%, 4 wt%, 4.5 wt%, 5 wt%, 5.5 wt%, 6 wt%, 6.5 wt%, 7 wt%, 7.5 wt%, 8 wt%, 8.5 wt%, 9 wt%, 9.5 wt%, 10 wt%, 10.5 wt%, 11 wt%, 11.5 wt%, 12 wt%, 12.5 wt%, 13 wt%, 13.5 wt%, 14 wt%, 14.5 wt%, 15 wt%, 15.5 wt%, 16 wt%, 16.5wt%, 17 wt%, 17.5 wt%, 18 wt%, 18.5 wt%, 19 wt%, 19.5 wt% or 20 wt%. Additionally, the content of the antifungal agent may range from one or more of the above values to one or less of the above values.

For example, the content range of the antifungal agent can be set to 0.1 wt% to 18 wt%, 0.5 wt% to 17 wt%, 1 wt% to 16 wt%, 2 wt% to 15 wt%, 3 wt% to 14 wt%, 4 wt% to 13 wt%, 5 wt% to 20 wt%, 8 wt% to 20 wt%, 10 wt% to 16 wt%, 1 wt% to 10 wt%, 5 wt% to 15 wt% or 0.1 wt% to 20 wt%.

Additionally, the content of the antifungal agent may be applied at a level higher than one of the above values, or may also be applied at a level lower than one of the above values. For example, the content of the antifungal agent may be applied as 0.1 wt% or more, 0.5 wt% or more, 1 wt% or more, 2 wt% or more, 3 wt% or more, 4 wt% or more, 5 wt% or more, 6 wt% or more, 7 wt% or more or 8 wt% or more, or may be applied as 20 wt% or less, 19 wt% or less, 18 wt% or less, 17 wt% or less, 16 wt% or less, 15 wt% or less, 14 wt% or less, 13 wt% or less, 12 wt% or less, 11 wt% or less or 10 wt% or less.

In one embodiment, the content of antifungal agent may be applied in an amount effective for the prevention or treatment of skin tinea and nail tinea. The effective dose level for the prevention or treatment of skin tinea and nail tinea may be determined by factors including the type and severity of the patient's disease, the activity of the drug, the sensitivity to the drug, the time of administration, the route of administration, the rate of excretion, the duration of treatment, concurrent medications, and other factors well known in the medical field. Taking all of the above factors into consideration, it is important to administer the amount that can achieve the maximum effect with the minimum amount without side effects, and this can be easily determined by those skilled in the art.

In one embodiment, the antifungal agent is a chemical substance that exhibits microbicidal activity against Trichophyton rubrum, Trichophyton mentagrophytes, Candida albicans, etc., and the type of antifungal agent is not limited to a specific type as long as it exhibits antimicrobial activity against the aforementioned fungi.

In one embodiment, the antifungal agent may include at least one selected from the group consisting of azole and oxaborole antimicrobial agents, such as luriconazole, luriconazole salt, efinaconazole, efinaconazole salt, tavaborole, and tavaborole salt.

In one specific example, the structural formula of luriconazole can be represented by the following Chemical Formula 1.

[Chemical Formula 1]

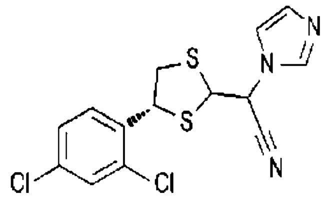

In addition, in one specific example, the luriconazole salt may be applied as at least one selected from the group consisting of an inorganic ionic salt, an inorganic acid salt, an organic acid salt, a sulfonate salt, an amino acid salt, and an amine salt as a pharmaceutically acceptable salt of luriconazole.

In one specific example, the structural formula of efinaconazole can be represented by the following Chemical Formula 2.

[Chemical Formula 2]

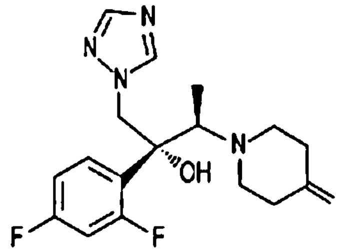

In addition, in one specific example, the efinaconazole salt may be applied as at least one selected from the group consisting of an inorganic ionic salt, an inorganic acid salt, an organic acid salt, a sulfonate salt, an amino acid salt, and an amine salt as a pharmaceutically acceptable salt of efinaconazole.

In one specific example, the structural formula of tavaborole can be represented by the following Chemical Formula 3.

[Chemical Formula 3]

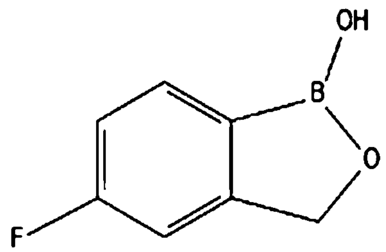

In addition, in one specific example, the tavaborole salt may be applied as at least one selected from the group consisting of an inorganic ionic salt, an inorganic acid salt, an organic acid salt, a sulfonate salt, an amino acid salt, and an amine salt as a pharmaceutically acceptable salt of tavaborole.

In one embodiment, a crystal growth inhibitor may inhibit the crystallization of a specific component included in a pharmaceutical composition. For example, a crystal growth inhibitor may prevent an antifungal agent included in the composition from crystallizing.

In one embodiment, the content of the crystal growth inhibitor may be 0.1 to 70 wt% based on the total weight of the composition. As a specific example, the content of crystal growth inhibitor may be set to 0.1 wt%, 0.2 wt%, 0.3 wt%, 0.4 wt%, 0.5 wt%, 0.6 wt%, 0.7 wt%, 0.8 wt%, 0.9 wt%, 1 wt%, 1.5 wt%, 2 wt%, 2.5 wt%, 3 wt%, 3.5 wt%, 4 wt%, 4.5 wt%, 5 wt%, 5.5 wt%, 6 wt%, 6.5 wt%, 7 wt%, 7.5 wt%, 8 wt%, 8.5wt%, 9 wt%, 9.5 wt%, 10 wt%, 10.5 wt%, 11 wt%, 11.5 wt%, 12 wt%, 12.5 wt%, 13 wt%, 13.5 wt%, 14 wt%, 14.5 wt%, 15 wt%, 15.5 wt%, 16 wt%, 16.5 wt%, 17 wt%, 17.5 wt%, 18 wt%, 18.5 wt%, 19 wt%, 19.5 wt%, 20 wt%, 25 wt%, 30 wt%, 35wt%, 40 wt%, 45 wt%, 50 wt%, 55 wt%, 60 wt%, 65 wt% or 70 wt%. Additionally, the content of the crystal growth inhibitor may range from one or more of the above values to one or less of the

above values.

For example, For example, the content range of the crystal growth inhibitor can be set to 0.1 wt% to 18 wt%, 0.5 wt% to 17 wt%, 1 wt% to 16 wt%, 2 wt% to 15 wt%, 3 wt% to 14 wt%, 4 wt% to 13 wt%, 5 wt% to 20 wt%, 8 wt% to 20 wt%, 10 wt% to 16 wt%, 1 wt% to 10 wt%, 5 wt% to 15 wt%, 0.1 wt% to 20 wt%, 10 wt% to 15 wt%, 10 wt% to 30 wt%, 10 wt% to 40 wt%, 20 wt% to 50 wt%, 30 wt% to 60 wt%, 40 wt% to 70 wt% or 0.1 wt% to 70 wt%.

Additionally, the content of the crystal growth inhibitor may be applied at a level higher than one of the above values, or may be applied at a level lower than one of the above values. For example, the content of the crystal growth inhibitor may be applied as 0.1 wt% or more, 0.5 wt% or more, 1 wt% or more, 2 wt% or more, 3 wt% or more, 4 wt% or more, 5 wt% or more, 6 wt% or more, 7 wt% or more, 8 wt% or more, 9 wt% or more, 10 wt% or more or 15 wt% or more, or may be applied as 70 wt% or less, 60 wt% or less, 50 wt% or less, 40 wt% or less, 30 wt% or less, 20 wt% or less or 10 wt% or less.

According to one embodiment, the crystal growth inhibitor may include 1,2-pentanediol and an ester compound. Further, in one embodiment, the ester compound may be at least one selected from the group consisting of acetyltributyl citrate, acetyltriethyl citrate, triethyl citrate, and diethyl carbonate.

In one embodiment, the weight ratio of 1,2-pentanediol and ester compound may be applied as 1:2. If the weight ratio between 1,2-pentanediol and ester compound exceeds 1:2, crystals may precipitate or sediment may occur during storage of the composition, which may negatively affect the penetration ability and antimicrobial activity of the composition.

In one embodiment, the keratin affinity inhibitor can inhibit the adsorption of the antifungal agent to keratin of the skin or nail. In one embodiment, the content of the keratin affinity inhibitor can be 0.01 to 30 wt% based on the total weight of the composition.

As a specific example, the content of the keratin affinity inhibitor can be set to 0.01 wt%, 0.05 wt%, 0.1 wt%, 0.2 wt%, 0.3 wt%, 0.4 wt%, 0.5 wt%, 0.6 wt%, 0.7 wt%, 0.8 wt%, 0.9 wt%, 1 wt%, 1.5 wt%, 2 wt%, 2.5 wt%, 3 wt%, 3.5 wt%, 4 wt%, 4.5 wt%, 5 wt%, 5.5 wt%, 6 wt%, 6.5 wt%, 7 wt%, 7.5 wt%, 8 wt%, 8.5 wt%, 9 wt%, 9.5 wt%, 10 wt%, 10.5 wt%, 11 wt%, 11.5 wt%, 12 wt%, 12.5 wt%, 13 wt%, 13.5 wt%, 14 wt%, 14.5 wt%, 15 wt%, 15.5 wt%, 16 wt%, 16.5 wt%, 17 wt%, 17.5 wt%, 18 wt%, 18.5 wt%, 19 wt%, 19.5 wt%, 20 wt%, 21 wt%, 22 wt%, 23 wt%, 24 wt%, 25 wt%, 26 wt%, 27 wt%, 28 wt%, 29 wt% or 30 wt%. Additionally, the content of the keratin affinity inhibitor may range from one or more of the above values to one or less of the above values.

For example, the content range of the keratin affinity inhibitor can be set to 0.1 wt% to 18 wt%, 0.5 wt% to 17 wt%, 1 wt% to 16 wt%, 2 wt% to 15 wt%, 3 wt% to 14 wt%, 4 wt% to 13 wt%, 5 wt% to 20 wt%, 8 wt% to 20 wt%, 10 wt% to 16 wt%, 1 wt% to 10 wt%, 5 wt% to 15 wt%, 0.1 wt% to 20 wt%, 10 wt% to 15 wt%, 10 wt% to 30 wt%, 20 wt% to 30 wt% or 0.01 wt% to 30 wt%.

Additionally, the content of the keratin affinity inhibitor may be applied at a level higher than one of the above values, or may be applied at a level lower than one of the above values. For example, the content of the keratin affinity inhibitor may be applied as 0.01 wt% or more, 0.05 wt% or more, 0.1 wt% or more, 0.5 wt% or more, 1 wt% or more, 2 wt% or more, 3 wt% or more, 4 wt% or more, 5 wt% or more, 6 wt% or more, 7 wt% or more, 8 wt% or more, 9 wt% or more, 10 wt% or more or 15 wt% or more, or may be applied as 30 wt% or less, 25 wt% or less, 20 wt% or less, 19 wt% or less, 18 wt% or less, 17 wt% or less, 16 wt% or less, 15 wt% or less, 14 wt% or less, 13 wt% or less, 12 wt% or less, 11 wt% or less or 10 wt% or less.

In one embodiment, the keratin affinity inhibitor may comprise cystamine dihydrochloride. In another embodiment, the keratin affinity inhibitor may further comprise at least one selected from lauryl choline, tricaprylin, and octenidine dihydrochloride. Depending on the implementation, the keratin affinity inhibitor may be used alone as one of the above-mentioned keratin affinity inhibitor components, or two or more may be used in combination.

Meanwhile, a pharmaceutical composition for the prevention or treatment of tinea according to one embodiment may further include a chelating agent. In one embodiment, the content of the chelating agent may be 0.0001 to 5 wt% based on the total weight of the composition.

As a specific example, the content of the chelating agent may be set to 0.0001 wt%, 0.001 wt%, 0.01 wt%, 0.1 wt%, 0.2 wt%, 0.3 wt%, 0.4 wt%, 0.5 wt%, 0.6 wt%, 0.7 wt%, 0.8 wt%, 0.9 wt%, 1 wt%, 1.5 wt%, 2 wt%, 2.5 wt%, 3 wt%, 3.5 wt%, 4 wt%, 4.5 wt% or 5 wt%. Additionally, the content of the chelating agent may range from one or more of the above values to one or less of the above values.

For example, the content range of the chelating agent can be set to 0.0001 wt% to 5 wt%, 0.0001 wt% to 4 wt%, 0.0001 wt% to 3 wt%, 0.0001 wt% to 2 wt%, 0.0001 wt% to 1 wt%, 0.0001 wt% to 0.5 wt%, 0.01 wt% to 5 wt%, 0.01 wt% to 1 wt%, 0.01 wt% to 0.1 wt% or 0.1 wt% to 5 wt%.

Additionally, the content of the chelating agent may be applied at a level higher than one of the above values, or may be applied at a level lower than one of the above values. For example, the content of the chelating agent may be applied as 0.0001 wt% or more, 0.001 wt% or more, 0.01 wt% or more, 1 wt% or more, 2 wt% or more, 3 wt% or more, 4 wt% or more or 4.5 wt% or more, or may be applied as 5 wt% or less, 4 wt% or less, 3 wt% or less, 2 wt% or less, 1 wt% or less, 0.5 wt% or less, 0.1 wt% or less, 0.01 wt% or less, 0.001 wt% or less, 0.005 wt% or less or 0.007 wt% or less.

In one embodiment, the chelating agent may comprise trisodium ethylenediamine disuccinate.

In one embodiment, the pharmaceutical composition for the prevention or treatment of tinea may further

comprise an additive. According to one embodiment, the additive may comprise at least one selected from a vehicle, an antioxidant, and a pH adjuster.

In one embodiment, the vehicle is an additive capable of maintaining the shape of the composition. According to one embodiment, the vehicle may be applied as at least one selected from the group consisting of purified water, glycerin, mineral oil, lanolin oil, sorbitol, ethanol, isopropyl alcohol, acetone, ethyl acetate, and mixtures thereof. Preferably, purified water, ethanol, isopropyl alcohol, glycerin, or the like may be used as the vehicle. In one embodiment, the content of the vehicle may be set in the range of 0.1 to 90 wt% based on the total weight of the composition.

As a specific example, the content of the vehicle can be set to 0.1 wt%, 0.2 wt%, 0.3 wt%, 0.4 wt%, 0.5 wt%, 0.6 wt%, 0.7 wt%, 0.8 wt%, 0.9 wt%, 1 wt%, 1.5 wt%, 2 wt%, 2.5 wt%, 3 wt%, 3.5 wt%, 4 wt%, 4.5 wt%, 5 wt%, 5.5 wt%, 6 wt%, 6.5 wt%, 7 wt%, 7.5 wt%, 8 wt%, 8.5 wt%, 9 wt%, 9.5wt%, 10 wt%, 10.5 wt%, 11 wt%, 11.5 wt%, 12 wt%, 12.5 wt%, 13 wt%, 13.5 wt%, 14 wt%, 14.5 wt%, 15 wt%, 15.5 wt%, 16 wt%, 16.5 wt%, 17 wt%, 17.5 wt%, 18 wt%, 18.5 wt%, 19 wt%, 19.5 wt%, 20 wt%, 25 wt%, 30 wt%, 35 wt%, 40 wt%, 45 wt%, 50 wt%, 55 wt%, 60 wt%, 65 wt%, 70 wt%, 75 wt%, 80 wt%, 85 wt% or 90 wt%.

In one embodiment, the antioxidant may be at least one selected from the group consisting of butylhydroxytoluene, butylhydroxyanisole, ferulic acid, ascorbic acid, and tocopherol. Preferably, butylhydroxytoluene may be used as the antioxidant. In one embodiment, the content of the antioxidant may be set in the range of 0.0001 to 1 wt% based on the total weight of the composition.

As a specific example, the content of the antioxidant can be set to 0.0001 wt%, 0.001 wt%, 0.01 wt%, 0.1 wt%, 0.2 wt%, 0.3 wt%, 0.4 wt%, 0.5 wt%, 0.6 wt%, 0.7 wt%, 0.8 wt%, 0.9 wt% or 1 wt%.

In one embodiment, the pH adjuster may be at least one selected from the group consisting of citric acid, pentetic acid, tartaric acid, and succinic acid, and preferably, citric acid may be used as the pH adjuster. In one embodiment, the content of the pH adjuster may be set in the range of 0.0001 to 5 wt% based on the total weight of the composition.

As a specific example, the content of the pH adjuster can be set to 0.0001 wt%, 0.001 wt%, 0.01 wt%, 0.1 wt%, 0.2 wt%, 0.3 wt%, 0.4 wt%, 0.5 wt%, 0.6 wt%, 0.7 wt%, 0.8 wt%, 0.9 wt%, 1 wt%, 2 wt%, 3 wt%, 4 wt% or 5 wt%.

According to one embodiment, the pharmaceutical composition for the prevention or treatment of tinea may be formulated as at least one formulation selected from a liquid, an aerosol, a stick, and a semi-solid preparation. In one embodiment, the semi-solid preparation may include at least one selected from a gel, a lotion, a cream, and an ointment.

The pH of the pharmaceutical composition for the prevention or treatment of tinea according to one embodiment may be applied as 2 to 10. As a specific example, the pH of the pharmaceutical composition for the prevention or treatment of tinea may be set to 2, 2.5, 3, 3.5, 4, 4.5, 5, 5.5, 6, 6.5, 7, 7.5, 8, 8.5, 9, 9.5, or 10. Additionally, the pH of the pharmaceutical composition for the prevention or treatment of tinea according to one embodiment may range from one or more of the above values to one or less of the above values. For example, the pH of the pharmaceutical composition may be in a range of 2 to 10, 4 to 10, 5 to 9.5, 6 to 8, or 7 to 8.

And, the density of the pharmaceutical composition for the prevention or treatment of tinea according to one embodiment can be applied as 0.5 to 2g/$cm^3$. As a specific example, the density of the pharmaceutical composition for the prevention or treatment of tinea can be applied as 0.5g/$cm^3$, 0.6g/$cm^3$, 0.7g/$cm^3$, 0.8g/$cm^3$, 0.9g/$cm^3$, 1g/$cm^3$, 1.1g/$cm^3$, 1.2g/$cm^3$, 1.3g/$cm^3$, 1.4g/$cm^3$, 1.5g/$cm^3$, 1.6g/$cm^3$, 1.7g/$cm^3$, 1.8g/$cm^3$, 1.9g/$cm^3$ or 2g/$cm^3$.

Additionally, in one embodiment, the density of the pharmaceutical composition of the topical preparation containing the antifungal agent as an active ingredient may range from one or more of the above values to one or less of the above values. For example, the density range of the pharmaceutical composition can be set to 0.5 to 2g/$cm^3$, 0.8 to 2g/$cm^3$, 1.2 to 1.8g/$cm^3$, 1.5 to 1.7g/$cm^3$ or 1.5 to 2g/$cm^3$.

In one embodiment, the moisture content of the pharmaceutical composition for the prevention or treatment of tinea is measured by the Karl Fischer method, and the moisture content of the composition measured by the Karl Fischer method can be 0.1 to 90 wt%. As a specific example, the moisture content of the pharmaceutical composition for the prevention or treatment of tinea may be applied as 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89% or 90%.

Additionally, the moisture content of the pharmaceutical composition of the topical preparation containing an antifungal agent as an active ingredient may range from one or more of the above values to one or less of the above values. For example, the moisture content range of the pharmaceutical composition can be set to 0.1 to 90%, 0.5 to 80%, 1 to 70%, 2 to 5%, 3 to 5%, 5 to 85%, 7 to 60%, 8 to 55%, 9 to 50%, 10 to 45%, 10 to 20%, 10 to 15%, 10 to 13%, 15 to 40% or 20 to 35%.

In one embodiment, the surface tension of the pharmaceutical composition for the prevention or treatment of tinea may be 1 to 76 dyn/cm. As a specific example, the surface tension of the pharmaceutical composition for the prevention or treatment of tinea may be applied as 1dyn/cm, 2dyn/cm, 3dyn/cm, 4dyn/cm, 5dyn/cm, 6dyn/cm, 7dyn/cm, 8dyn/cm, 9dyn/cm, 10dyn/cm, 11dyn/cm, 12dyn/cm, 13dyn/cm, 14dyn/cm, 15dyn/cm, 16dyn/cm, 17dyn/cm, 18dyn/cm,

19dyn/cm, 20dyn/cm, 21dyn/cm, 22dyn/ cm, 23dyn/cm, 24dyn/cm, 25dyn/cm, 26dyn/cm, 27dyn/cm, 28dyn/cm, 29dyn/cm, 30dyn/cm, 31dyn/cm, 32dyn/cm, 33dyn/cm, 34dyn/cm, 35dyn/cm, 36dyn/cm, 37dyn/cm, 38dyn/cm, 39dyn/cm, 40dyn/cm, 41dyn/cm, 42dyn/cm, 43dyn/cm, 44dyn/cm, 45dyn/cm, 46dyn/cm, 47dyn/cm, 48dyn/cm, 49dyn/cm, 50dyn/cm, 51dyn/cm, 52dyn/cm, 53dyn/cm, 54dyn/cm, 55dyn/cm, 56dyn/cm, 57dyn/cm, 58dyn/cm, 59dyn/cm, 60dyn/cm, 61dyn/cm, 62dyn/cm, 63dyn/cm, 64dyn/cm, 65dyn/cm, 66dyn/cm, 67dyn/cm, 68dyn/cm, 69dyn/cm, 70dyn/cm, 71dyn/cm, 72dyn/cm, 73dyn/cm, 74dyn/cm, 75dyn/cm or 76dyn/cm at 25°C.

Additionally, the surface tension of the pharmaceutical composition of the topical preparation containing an antifungal agent as an active ingredient may range from one or more of the above values to one or less of the above values. For example, the surface tension range of pharmaceutical composition may be set to 1dyn/cm to 76dyn/cm, 5dyn/cm to 60dyn/cm, 10dyn/cm to 50dyn/cm, 15dyn/cm to 65dyn/cm, 20dyn/cm to 60dyn/cm, 10dyn/cm to 40dyn/cm, 15dyn/cm to 30dyn/cm, 25dyn/cm to 55dyn/cm, 30dyn/cm to 50dyn/cm, 4ldyn/cm to 50dyn/cm, 42dyn/cm to 50dyn/cm, 43dyn/cm to 50dyn/cm, 44dyn/cm to 50dyn/cm, 45dyn/cm to 50dyn/cm, 41dyn/cm to 45dyn/cm, 4ldyn/cm to 44dyn/cm, 4ldyn/cm to 43dyn/cm or 50dyn/cm to 76dyn/cm.

According to one embodiment, when applied topically to the pharmaceutical composition for the prevention or treatment of tinea, the daily application amount may be 0.01 to 60g/day. For example, when the pharmaceutical composition is applied externally, the daily dosage may be applied as 0.01g/day, 0.02g/day, 0.03g/day, 0.04g/day, 0.05g/day, 0.06g/ day, 0.07g/day, 0.08g/day, 0.09g/day, 0.1g/day, 0.2g/day, 0.3g/day, 0.4g/day, 0.5g/day, 0.6g/day, 0.7g/day, 0.8g/day, 0.9g/day, 1g/day, 2g/day, 3g/day, 4g/day, 5g/day, 6g/day, 7g/day, 8g/day, 9g/day, 10g/day, 11g/day, 12g/day, 13g/day, 14g/day, 15g/day, 16g/day, 17g/day, 18g/day, 19g/day, 20g/day, 21g/day, 22g/day, 23g/day, 24g/day, 25g/day, 26g/day, 27g/day, 28g/day, 29g/day, 30g/day, 31g/day, 32g/day, 33g/day, 34g/day, 35g/day, 36g/day, 37g/day, 38g/day, 39g/day, 40g/day, 41g/day, 42g/day, 43g/day, 44g/day, 45g/day, 46g/day, 47g/day, 48g/day, 49g/day, 50g/day, 51g/day, 52g/day, 53g/day, 54g/day, 55g/day, 56g/day, 57g/day, 58g/day, 59g/day or 60g/day.

In addition, when the pharmaceutical composition of a topical preparation containing an antifungal agent as an active ingredient is applied topically, the daily application amount may range from one or more of the above values to one or less of the above values. For example, when the pharmaceutical composition is applied topically, the daily application amount range can be set to 0.01 to 60g/day, 0.1 to 60g/day, 0.01 to 1g/day, 0.01 to 0.5g/day, 0.05 to 0.1g/day, 1 to 60g/day, 5 to 50g/day, 10 to 40g/day, 15 to 35g/day or 20 to 30g/day.

And, in one embodiment, the pharmaceutical composition may be prepared as a topical preparation, and when applied topically, a method of applying, spraying, or attaching an appropriate amount of the composition to the treatment area of the patient (e.g., nail, skin, etc.) may be used.

Meanwhile, a pharmaceutical composition for the prevention or treatment of tinea according to one embodiment may not include cyclomethicone, methylpyrrolidone and ethylenediamine tetraacetic acid salt. That is, according to one embodiment, the pharmaceutical composition for the prevention or treatment of tinea does not contain cyclomethicone, methylpyrrolidone and ethylenediamine tetraacetic acid salt, thereby fundamentally eliminating the harmful effects caused by these ingredients.

According to one embodiment, the color of the pharmaceutical composition of the topical preparation containing an antifungal agent as an active ingredient may be colorless or pale yellow. In addition, the pharmaceutical composition according to one embodiment does not crystallize even after being stored at -10°C for at least 2 weeks, and does not crystallize even after being applied to the skin or nail and being stored at room temperature (25°C) for 24 hours.

The present invention is described in more detail below through specific manufacturing examples. The following manufacturing examples are merely examples intended to aid understanding of the present invention and are not intended to limit or restrict the scope of the present invention.

<u>Control Examples 1 to 3, Comparative Examples 1 to 3, Manufacturing Examples 1 to 15: Preparation of topical solution</u>

A topical solution containing an antifungal agent (luriconazole, efinaconazole, tavaborole) as an active ingredient was prepared according to the procedure below, and the components and composition of each topical solution are as described in Tables 1, 2 and 3 below.

1. Preparation of Solution A: After dissolving antifungal agents (luriconazole, efinaconazole, tavaborole) in ethanol, 1,2-pentanediol, acetyltributyl citrate, cystamine dihydrochloride, butylhydroxytoluene, and glycerin were sequentially added and dissolved at room temperature (25°C) to prepare Solution A.

2. Preparation of Solution B: Solution B was prepared by sequentially adding citric acid and trisodium ethylenediamine disuccinate to purified water and dissolving them.

3. Solution B was added to Solution A, mixed and dissolved, and then filtered through a 0.45 $\mu$m filter to prepare a transparent topical solution.

4. The pH of the manufactured topical solution was measured and found to be within the range of 4.5 to 7.5, and the surface tension of the manufactured topical solution was measured and found to be within the range of 25 to 50 dyn/cm at 25°C.

[Table 1]

| Component | Manufacturing Example (wt%) | | | | | | |
|---|---|---|---|---|---|---|---|
| | Cont. Ex. 1 | Comp. Ex. 1 | Manuf . Ex. 1 | Manuf . Ex. 2 | Manuf. Ex. 3 | Manuf . Ex. 4 | Manuf. Ex. 5 |
| Luriconazole | 5.00 | 5.00 | 5.00 | 5.00 | 5.00 | 5.00 | 5.00 |
| 1,2-Pentanediol | - | 10.00 | 10.00 | 10,00 | 10.00 | 10.00 | 10.00 |
| Acetyltributyl citrate | - | 20.00 | 5.00 | 10.00 | 20.00 | 30.00 | 40.00 |
| Cystamine dihydrochloride | - | - | 10.00 | 10,00 | 10,00 | 10.00 | 10.00 |
| Butylhydroxy toluene | 0.10 | 0.10 | 0.10 | 0.10 | 0.10 | 0.10 | 0.10 |
| Citric acid | 0.20 | 0.20 | 0.20 | 0.20 | 0.20 | 0.20 | 0.20 |
| Trisodium ethylenediamine disuccinate | 0.10 | 0.10 | 0.10 | 0.10 | 0.10 | 0.10 | 0.10 |
| Glycerin | 5.00 | 5.00 | 5.00 | 5.00 | 5.00 | 5.00 | 5.00 |
| Ethanol | 87.60 | 57.60 | 62.60 | 57.60 | 47.60 | 37.60 | 27.60 |
| Purified water | 2.00 | 2.00 | 2.00 | 2.00 | 2.00 | 2.00 | 2.00 |
| Total (%) | 100.00 | 100.00 | 100.00 | 100.00 | 100.00 | 100.00 | 100.00 |

[Table 2]

| Component | Manufacturing Example (wt%) | | | | | | |
|---|---|---|---|---|---|---|---|
| | Cont. Ex. 2 | Comp. Ex. 2 | Manuf . Ex. 6 | Manuf . Ex. 7 | Manuf. Ex. 8 | Manuf . Ex. 9 | Manuf. Ex. 10 |
| Efinaconazole | 10.00 | 10.00 | 10.00 | 10.00 | 10.00 | 10.00 | 10.00 |
| 1,2-Pentanediol | - | 10.00 | 10.00 | 10,00 | 10.00 | 10.00 | 10.00 |
| Acetyltributyl citrate | - | 20.00 | 5.00 | 10.00 | 20.00 | 30.00 | 40.00 |
| Cystamine dihydrochloride | - | - | 10.00 | 10,00 | 10,00 | 10.00 | 10.00 |
| Butylhydroxy toluene | 0.10 | 0.10 | 0.10 | 0.10 | 0.10 | 0.10 | 0.10 |
| Citric acid | 0.20 | 0.20 | 0.20 | 0.20 | 0.20 | 0.20 | 0.20 |
| Trisodium ethylenediamine disuccinate | 0.10 | 0.10 | 0.10 | 0.10 | 0.10 | 0.10 | 0.10 |
| Glycerin | 5.00 | 5.00 | 5.00 | 5.00 | 5.00 | 5.00 | 5.00 |
| Ethanol | 82.60 | 52.60 | 57.60 | 52.60 | 42.60 | 32.60 | 22. 60 |
| Purified water | 2.00 | 2.00 | 2.00 | 2.00 | 2.00 | 2.00 | 2.00 |
| Total (%) | 100.00 | 100.00 | 100.00 | 100.00 | 100.00 | 100.00 | 100.00 |

[Table 3]

| Component | Manufacturing Example (wt%) | | | | | | |
|---|---|---|---|---|---|---|---|
| | Cont. Ex. 3 | Comp. Ex. 3 | Manuf . Ex. 11 | Manuf . Ex. 12 | Manuf. Ex. 13 | Manuf . Ex. 14 | Manuf. Ex. 15 |
| Tavaborole | 5.00 | 5.00 | 5.00 | 5.00 | 5.00 | 5.00 | 5.00 |
| 1,2-Pentanediol | - | 10.00 | 10.00 | 10,00 | 10.00 | 10.00 | 10.00 |
| Acetyltributyl citrate | - | 20.00 | 5.00 | 10.00 | 20.00 | 30.00 | 40.00 |

(continued)

| Component | Manufacturing Example (wt%) | | | | | | |
|---|---|---|---|---|---|---|---|
| | Cont. Ex. 3 | Comp. Ex. 3 | Manuf . Ex. 11 | Manuf . Ex. 12 | Manuf. Ex. 13 | Manuf . Ex. 14 | Manuf. Ex. 15 |
| Cystamine dihydrochloride | - | - | 10.00 | 10,00 | 10,00 | 10.00 | 10.00 |
| Butylhydroxy toluene | 0.10 | 0.10 | 0.10 | 0.10 | 0.10 | 0.10 | 0.10 |
| Citric acid | 0.20 | 0.20 | 0.20 | 0.20 | 0.20 | 0.20 | 0.20 |
| Trisodium ethylenediamine disuccinate | 0.10 | 0.10 | 0.10 | 0.10 | 0.10 | 0.10 | 0.10 |
| Glycerin | 5.00 | 5.00 | 5.00 | 5.00 | 5.00 | 5.00 | 5.00 |
| Ethanol | 87.60 | 57.60 | 62.60 | 57.60 | 47.60 | 37.60 | 27.60 |
| Purified water | 2.00 | 2.00 | 2.00 | 2.00 | 2.00 | 2.00 | 2.00 |
| Total (%) | 100.00 | 100.00 | 100.00 | 100.00 | 100.00 | 100.00 | 100.00 |

Test Example 1. Test for evaluation of ability to suppress crystal precipitation at low temperature

Each sample of Control Examples 1 to 3, Comparative Examples 1 to 3 and Manufacturing Examples 1 to 15 was added to a 20 ml vial at 10 ml each, stored in a low-temperature freezer at -10°C for 2 weeks, and then the crystal and precipitate formation state was observed with the naked eye at room temperature (25°C). The results are shown in Table 4.

[Table 4]

| Cont. Ex. 1 | Comp. Ex. 1 | Manuf . Ex. 1 | Manuf . Ex. 2 | Manuf . Ex. 3 | Manuf . Ex. 4 | Manuf. Ex. 5 |
|---|---|---|---|---|---|---|
| Crystal /Precip. -> A lot | Crystal /Precip. - > None (Good) | Crystal /Precip. -> A lot | Crystal /Precip. -> Little | Crystal /Precip. -> None (Good) | Crystal /Precip. -> Fine | Crystal /Precip. -> A lot |
| Cont. Ex. 2 | Comp. Ex. 2 | Manuf . Ex. 6 | Manuf . Ex. 7 | Manuf . Ex. 8 | Manuf . Ex. 9 | Manuf. Ex. 10 |
| Crystal /Precip. -> A lot | Crystal /Precip. -> None (Good) | Crystal /Precip. -> A lot | Crystal /Precip. -> Little | Crystal /Precip. -> None (Good) | Crystal /Precip. -> Fine | Crystal /Precip. -> A lot |
| Cont. Ex. 3 | Comp. Ex. 3 | Manuf . Ex. 11 | Manuf . Ex. 12 | Manuf . Ex. 13 | Manuf . Ex. 14 | Manuf. Ex. 15 |
| Crystal /Precip. -> A lot | Crystal /Precip. -> None (Good) | Crystal /Precip. -> A lot | Crystal /Precip. -> Fine | Crystal /Precip. -> None (Good) | Crystal /Precip. -> Little | Crystal /Precip. -> A lot |

Referring to the results in Table 4, in the case of Comparative Examples 1, 2 and 3 and Manufacturing Examples 3, 8 and 13, where the weight ratio of 1,2-pentanediol and acetyltributyl citrate as crystal growth inhibitors was 1:2 and the content of crystal growth inhibitor was 30 wt% of the total weight of the topical solution, it was confirmed that no crystal precipitation phenomenon occurred at all even after storage at -10°C for 2 weeks.

Test Example 2. Test for evaluation of ability to suppress crystal precipitation during drying after application of topical preparation

Each sample of Control Examples 1 to 3, Comparative Examples 1 to 3, and Manufacturing Examples 1 to 15 was dropped in an amount of 0.5 ml onto a slide glass measuring 26 mm in width x 76 mm in height, applied with a cotton swab, and observed with the naked eye after 24 hours at room temperature (25°C). The results are shown in Table 5.

[Table 5]

| | | | | | | |
|---|---|---|---|---|---|---|
| Cont. Ex. 1 | Comp. Ex. 1 | Manuf. Ex. 1 | Manuf . Ex. 2 | Manuf . Ex. 3 | Manuf. Ex. 4 | Manuf. Ex. 5 |
| Crystal ->A lot | Crystal ->None (Good) | Crystal ->A lot | Crystal ->A lot | Crystal ->None (Good) | Crystal ->Little | Crystal ->A lot |
| Cont. Ex. 2 | Comp. Ex. 2 | Manuf. Ex. 6 | Manuf . Ex. 1 | Manuf . Ex. 8 | Manuf. Ex. 9 | Manuf. Ex. 10 |
| Crystal ->A lot | Crystal ->None (Good) | Crystal ->A lot | Crystal ->A lot | Crystal ->None (Good) | Crystal ->Little | Crystal ->A lot |
| Cont. Ex. 3 | Comp. Ex. 3 | Manuf. Ex. 11 | Manuf . Ex. 12 | Manuf . Ex. 13 | Manuf. Ex. 14 | Manuf. Ex. 15 |
| Crystal ->A lot | Crystal ->None (Good) | Crystal ->A lot | Crystal ->Little | Crystal ->None (Good) | Crystal ->A lot | Crystal ->A lot |

Referring to the results in Table 5, in the case of Comparative Examples 1, 2 and 3 and Manufacturing Examples 3, 8 and 13, where the weight ratio of 1,2-pentanediol and acetyltributyl citrate as crystal growth inhibitors was 1:2 and the content of crystal growth inhibitor was 30 wt% of the total weight of the topical solution, it was confirmed that no crystal precipitation phenomenon occurred at all even after 24 hours at room temperature after applying the topical preparation to the slide glass.

Test Example 3. Susceptibility testing for antimicrobial activity evaluation

Typically, clinical treatment outcomes can be predicted through susceptibility testing of antifungal agent. The test strains used for antimicrobial susceptibility testing are representative causative organisms causing superficial fungal infections, including Trichophyton rubrum, Trichophyton mentagrophytes, and Candida albicans. To evaluate the antimicrobial activity against the three types of microorganisms mentioned above, this experiment was conducted using the tube dilution technique. Antimicrobial activity was evaluated by comparing the minimum growth inhibition concentration ($MIC_{90}$) of Control Examples 1, 2 and 3, Comparative Examples 1, 2 and 3, and Manufacturing Examples 3, 8 and 13 for each microorganism, and the results are shown in Table 6 below. The minimum growth inhibition concentration test was performed according to the classification of the CLSI (Clinical and Laboratory Standards Institute) standards. Trichophyton rubrum and Trichophyton mentagrophytes as fungi were tested using the CLSI M38-A2:2008 test method, and Candida albicans as yeast was tested using the CLSI M27-A3:2008 test method, as follows.

1) Preculture of test microorganism

① Trichophyton rubrum, Trichophyton mentagrophytes: The cultures were spread on OA (OA; Oatmeal 6%, Agar 1.25%, pH 6.0) medium and cultured at 30±2°C for 4 to 5 days.

② Candida albicans: The culture was inoculated onto Sabouraud Dextrose Agar (SDA) medium and cultured at 35±2°C for 20 to 24 hours.

2) Preparation of test microorganism solutions and spore suspensions

① Trichophyton rubrum, Trichophyton mentagrophytes: After buffering RPMI 1640 medium (Gibco company) with 0.165 mol/L 3-N-morpholinolinopropane sulfonic acid (MOPS), a spore suspension of (2-3) x $10^3$ CFU/ml was prepared and used as a test microorganism solution.
② Candida albicans: After buffering RPMI 1640 medium (Gibco company) with 0.165 mol/L 3-N-morpholinopropane sulfonic acid, a spore suspension of 5.0 x $10^2$ to 2.5 x $10^3$ CFU/ml was prepared and used as a test microorganism solution.

3) Inoculation of test microorganism solution

① Trichophyton rubrum, Trichophyton mentagrophytes: 1 ml of the test microorganism solution was inoculated into each well containing the test solution for each concentration. A well containing 0.2 ml of the solution (Control Example 1, 2 and 3, Comparative Example 1, 2 and 3, Manufacturing Example 3, 8 and 13) in RPMI 1640 (buffered with MOPS 0.165 mol/L) medium was used as a negative control, and a well containing 0.1 ml of the

solution (Control Example 1, 2 and 3, Comparative Example 1, 2 and 3, Manufacturing Example 3, 8 and 13) and 0.1 ml of the test microorganism solution in RPMI 1640 (buffered with MOPS 0.165 mol/L) medium was used as a positive control. The tubes that had been treated with the test microorganism solution were cultured at 35±2°C for 46 to 50 hours.

② Candida albicans: 0.9 ml of the test microorganism solution was inoculated into each well containing the test solution for each concentration. A well containing 1.0 ml of the solution (Control Example 1, 2 and 3, Comparative Example 1, 2 and 3, Manufacturing Example 3, 8 and 13) in RPMI 1640 (buffered with MOPS 0.165 mol/L) medium was used as a negative control, and a well containing 0.1 ml of the solution (Control Example 1, 2 and 3, Comparative Example 1, 2 and 3, Manufacturing Example 3, 8 and 13) and 0.9 ml of the test microorganism solution in RPMI 1640 (buffered with MOPS 0.165 mol/L) medium was used as a positive control. The tubes that had been treated with the test microorganism solution were cultured at 35±2°C for 24 to 48 hours.

4) Results Analysis

After incubation, microbial growth was visually observed in each tube. The minimum concentration at which no microbial growth occurred was defined as the minimum growth inhibitory concentration ($MIC_{90}$). After incubation, no growth should be observed in the negative control group, and microbial growth should be observed in the positive control group. The results obtained using the above antimicrobial activity test method are presented in Table 6.

[Table 6]

| Strain | Minimum growth inhibitory concentration ($MIC_{90}$, ug/ml) | | | | | | | | |
|---|---|---|---|---|---|---|---|---|---|
| | Cont. Ex. 1 | Comp. Ex. 1 | Manuf . Ex. 3 | Cont. Ex. 2 | Comp. Ex. 2 | Manuf. Ex. 8 | Cont. Ex. 3 | Comp. Ex. 3 | Manuf. Ex. 13 |
| Trichophyton rubrum (ATCC 28188) | 0.016 | 0.010 | 0.005 | 0.021 | 0.013 | 0.008 | 2.141 | 1.878 | 0.816 |
| Trichophyton mentagrophytes (ATCC 18748) | 0.031 | 0.016 | 0.007 | 0.036 | 0.022 | 0.010 | 4.325 | 3.543 | 2.026 |
| Candida albicans (ATCC 1023) | 0.326 | 0.297 | 0.132 | 0.785 | 0.614 | 0.324 | 5.416 | 4.766 | 2.582 |

As can be seen from the results in Table 6, for Trichophyton rubrum, Manufacturing Examples 3, 8 and 13 were 2.6 to 3.2 times better than Control Examples 1, 2 and 3, and 1.6 to 2.3 times better than Comparative Examples 1, 2 and 3. For Trichophyton mentagrophytes, Manufacturing Examples 3, 8 and 13 were 2.1 to 4.4 times better than Control Examples 1, 2 and 3, and 1.7 to 2.3 times better than Comparative Examples 1, 2 and 3. For Candida albicans, Manufacturing Examples 3, 8 and 13 were 2.1 to 2.5 times better than Control Examples 1, 2 and 3, and 1.8 to 2.3 times better than Comparative Examples 1, 2 and 3. Therefore, the pharmaceutical composition according to the Manufacturing Examples shows good susceptibility test results against the causative organisms causing tinea, so it can be predicted that it will not only be able to treat the aforementioned causative organisms simultaneously, but will also show excellent clinical treatment results.

<Method for analyzing the content of antifungal agent>

In the following Test Examples 4, 5 and 6, content analysis of luriconazole was performed using a UV-Visible Spectrometer under the conditions below.

* Content analysis of luriconazole

- Apparatus: Mega-800 (Scinco company, Korea), double-beam method
- Measurement cell: 10 mm quartz cuvettes
- Solvent: Methanol
- Preparation of standard solution: Dissolve 50 mg of luriconazole standard in 30 ml of methanol, sonicate for 10 minutes, fill to 50 ml with methanol, and dilute 1 ml of this solution with 100 ml of methanol.
- Preparation of sample treatment solution: Accurately weigh approximately 1 gram of the solution to be used as the

sample, dissolve in 70 ml of methanol, sonicate for 10 minutes, fill to 100 ml with methanol, filter through Whatman filter paper, and dilute 5 ml of the filtered solution with 50 ml of methanol.

- Measurement procedure: Measure the absorbance of the standard solution and the sample treatment solution at 296 nm using a UV-Vis spectrophotometer, using the blank cell as a reference.

$$\text{** Luriconazole content (wt\%)} = \frac{\text{Absorbance of standard solution X Weight of standard (mg) X Purity of standard(\%) X (100-Drying loss of standard)(\%) X 100}}{\text{Absorbance of sample treatment solution X Weight of sample(mg) X 5}}$$

In the following Test Examples 4, 5 and 6, high-performance liquid chromatography (HPLC) was used to analyze the content of efinaconazole under the conditions below.

* Efinaconazole Content Analysis

- Apparatus: Agilent HOO (DAD); Time-dependent Concentration Gradient
- Column: Inertsil ODS3V (250 x 4.6 mm, 5.0 μm)
- Column Temperature: 40°C
- Mobile Phase: ⓐ Buffer Solution ⓑ Acetonitrile: Methanol (50:50 vol %)
- Flow Rate: 1.0 ml/min
- Detector: UV Detector
- Detection Wavelength: 210 nm
- Sample Injection Volume: 10.0 μl
- Sample Concentration: 0.3 mg/ml

* ⓐ Preparation of buffer solution: Dissolve 1.36 g of $KH_2PO_4$ and 2.16 g of 1-Octanesulfonic acid sodium in 1,000 ml of purified water, adjust the pH to 2.5, and then filter and degas through a 0.45 μ membrane filter.

$$\text{**Efinaconazole content (wt\%)} = \frac{\text{Area of efinaconazole in sample X Weight of standard(mg) X Purity of standard(\%)X(100- Drying loss of standard)(\%)X100}}{\text{Area of efinaconazole in standard X Weight of sample (mg)}}$$

In the following Test Examples 4, 5 and 6, the content analysis of tavaborole was performed using a UV-Visible Spectrometer under the conditions below.

* Tavaborole Content Analysis (wt%)

- Apparatus: Mega-800 (Scinco company, Korea), double-beam method
- Measurement Cell: 10 mm Quartz cuvettes
- Solvent: Methanol
- Preparation of standard solution: Dissolve 50 mg of tavaborole standard in 30 ml of methanol, sonicate for 10 minutes, fill to 50 ml with methanol, and dilute 1 ml of this solution with 100 ml of methanol.
- Preparation of sample treatment solution: Accurately weigh about 1 gram of the solution to be used as the sample, dissolve in 70 ml of methanol, sonicate for 10 minutes, fill to 100 ml with methanol, filter through Whatman filter paper, and dilute 5 ml of the filtered solution with 50 ml of methanol.
- Measurement procedure: Measure the absorbance of the standard solution and the sample treatment solution at 272 nm using a UV-Vis spectrophotometer, using the blank cell as a control.

$$\text{**Tavaborole content (wt\%)} = \frac{\text{Absorbance of standard solution X Weight of standard (mg) X Purity of standard(\%) X (100-Drying loss of standard)(\%) X 100}}{\text{Absorbance of sample treatment solution X Weight of sample(mg) X 5}}$$

Test Example 4. Evaluation of keratin affinity inhibition performance

After freezing the cow hooves with liquid nitrogen, they were ground into powder with a particle size of 5 mm or less, sterilized with ethylene oxide gas, and then sequentially washed and degreased with saline solution, distilled water, ethanol/ether (1/1, v/v%), and acetone, and dried to produce keratin powder. In a 30 ml glass vial, keratin powder and solutions of Control Examples 1 to 3, Comparative Examples 1 to 3, and Manufacturing Examples 3, 8, and 13 were mixed at a certain ratio (0.5 ml of solution per 1 g of keratin powder), shaken in a 37°C water bath for 2 hours, centrifuged at 1,500 rpm for 5 minutes, and the resulting supernatant was collected to analyze the concentration of the unadsorbed drug on keratin, and the adsorption rate and keratin affinity inhibition rate for Control Example were obtained as follows, and the results are shown in Table 7.

**Keratin adsorption rate (%) = {(added drug concentration - drug concentration in supernatant) / added drug concentration} x 100

[Table 7]

| Sample | Keratin adsorption rate (%) | Keratin affinity reduction rate (%) compared to Control Example |
|---|---|---|
| Cont. Ex. 1 | 95.2±1.4 | - |
| Comp. Ex. 1 | 91.8±0.3 | 3.4±1.1 |
| Manuf. Ex. 3 | 81.6±0.7 | 13.6+0.7 |
| Cont. Ex. 2 | 91.6±0.8 | - |
| Comp. Ex. 2 | 87.8±0.2 | 3.8±0.6 |
| Manuf. Ex. 8 | 77.3±0.5 | 14.3±0.3 |
| Cont. Ex. 3 | 96.3±1.2 | - |
| Comp. Ex. 3 | 92.9±0.9 | 3.4±0.3 |
| Manuf. Ex. 13 | 80.5±0.3 | 15.8±0.9 |

As shown in Table 7, the keratin affinity reduction rate (%) was 3.4% for Comparative Example 1 and 13.6% for Manufacturing Example 3 compared to Control Example 1, 3.8% for Comparative Example 2 and 14.3% for Manufacturing Example 8 compared to Control Example 2, and 3.4% for Comparative Example 3 and 15.8% for Manufacturing Example 13 compared to Control Example 3. That is, it can be seen that cystamine dihydrochloride included in the composition shows a very good effect as a keratin affinity inhibitor and thus plays an important role in improving drug permeability.

Test Example 5. Evaluation of skin permeability using human skin

The skin permeability of antifungal agents was evaluated using human skin and solutions according to Control Examples 1 to 3, Comparative Examples 1 to 3, and Manufacturing Examples 1 to 15 in a Franz Diffusion Cell. Human skin was prepared using HuSKin (Hans Biomed Co.), and after hydration for 2 hours, the hydrated skin was mounted on a Franz diffusion cell, 200 μl of each test sample was applied to the donor compartment, and 5 ml of phosphate-buffered saline (PBS) containing 1.0% (w/v) Tween 80 and 0.002% (w/v) kanamycin sulfate was filled into the acceptor compartment. The diffusion area was 1.13 $cm^2$. The sample volume was 200 μl for each measurement, and the same amount of acceptor compartment solution was replenished. The stirring speed was 600 rpm, the temperature was 37±0.5°C, and the number of repetitions per test was three. Sampling was performed every 24 and 48 hours. Each sample collected was analyzed according to the antifungal agent content analysis method described above, and the results are presented in Tables 8, 9, and 10.

[Table 8]

| Sample | 24 hour | 48 hour |
|---|---|---|
| | Permeate concentration of luriconazole (ug/ml) | Permeate concentration of luriconazole (ug/ml) |
| Cont. Ex. 1 | 22.15±1.65 | 31.55±2.14 |
| Comp. Ex. 1 | 44.56±1.23 | 65.12±1.53 |
| Manuf. Ex. 1 | 28.65±1.15 | 43.14±1.32 |
| Manuf. Ex. 2 | 37.26±1.54 | 54.34±2.54 |
| Manuf. Ex. 3 | 55.62±1.14 | 102.64±1.43 |
| Manuf. Ex. 4 | 39.15±2.43 | 52.26±1.54 |
| Manuf. Ex. 5 | 27.14±1.87 | 45.12±2.13 |

As shown in Table 8, Manufacturing Example 3 showed the highest permeate concentration, with permeate concentrations of 2.5 and 1.3 times for 24 hours and cumulative permeate concentrations of 3.3 and 1.6 times for 48 hours, compared to Control Example 1 and Comparative Example 1. Therefore, when the crystal growth inhibitor (1,2-pentanediol: acetyltributyl citrate weight ratio is 1:2) is contained at 30 wt% based on the total weight of the composition, and the keratin affinity inhibitor (cystamine dihydrochloride) is contained at 10 wt% based on the total weight of the composition, the treatment efficiency of tinea can be further improved.

[Table 9]

| Sample | 24 hour | 48 hour |
|---|---|---|
| | Permeate concentration of efinaconazole (ug/ml) | Permeate concentration of efinaconazole (ug/ml) |
| Cont. Ex. 2 | 36.26±1.04 | 49.75±2.25 |
| Comp. Ex. 2 | 64.23±1.43 | 120.76±1.32 |
| Manuf. Ex. 6 | 50.13±1.24 | 88.62±1.54 |
| Manuf. Ex. 7 | 57.58±1.16 | 97.54±2.14 |
| Manuf. Ex. 8 | 95.24±1.37 | 190.32±1.16 |
| Manuf. Ex. 9 | 54.12±2.12 | 91.46±1.12 |
| Manuf. Ex. 10 | 40.11±1.14 | 68.34±1.56 |

As shown in Table 9, Manufacturing Example 8 showed the highest permeate concentration, with permeate concentrations of 2.6 times and 1.5 times for 24 hours and cumulative permeate concentrations of 3.8 times and 1.6 times for 48 hours, compared to Control Example 2 and Comparative Example 2. Therefore, when the crystal growth inhibitor (weight ratio of 1,2-pentanediol: acetyltributyl citrate is 1:2) is contained at 30 wt% based on the total weight of the composition, and the keratin affinity inhibitor (cystamine dihydrochloride) is contained at 10 wt% based on the total weight of the composition, the treatment efficiency of tinea can be further improved.

[Table 10]

| Sample | 24 hour | 48 hour |
|---|---|---|
| | Permeate concentration of tavaborole (ug/ml) | Permeate concentration of tavaborole (ug/ml) |
| Cont. Ex. 3 | 25.22±1.78 | 39.45±2.11 |
| Comp. Ex. 3 | 54.18±0.94 | 82.34±1.25 |
| Manuf. Ex. 11 | 30.56±1.23 | 48.32±1.45 |
| Manuf. Ex. 12 | 45.21±2.35 | 61.25±1.27 |
| Manuf. Ex. 13 | 81.94±1.55 | 147.52±1.69 |
| Manuf. Ex. 14 | 47.12±2.15 | 66.24±1.18 |
| Manuf. Ex. 15 | 34. 62±1.38 | 50.11±2.04 |

As shown in Table 10, Manufacturing Example 13 showed the highest permeate concentration, with permeate concentrations of 3.2 times and 1.5 times for 24 hours and cumulative permeate concentrations of 3.7 times and 1.8 times for 48 hours, compared to Control Example 3 and Comparative Example 3. Therefore, when the crystal growth inhibitor (weight ratio of 1,2-pentanediol: acetyltributyl citrate is 1:2) is contained at 30 wt% based on the total weight of the composition, and the keratin affinity inhibitor (cystamine dihydrochloride) is contained at 10 wt% based on the total weight of the composition, the treatment efficiency of tinea can be further improved.

Test Example 6. Evaluation of human nail penetration

A nail cut from a healthy adult was obtained, washed with saline solution, and then hydrated in saline solution for 2 hours. The hydrated nail was mounted on a Franz diffusion cell, and then 100 μl of each sample (Control Examples 1 to 3, Comparative Examples 1 to 3, Manufacturing Examples 1 to 15) was applied to the donor compartment once a day for 10 days, and 200 μl of a sample was collected from the acceptor compartment. The acceptor compartment is filled with 5 ml of phosphate buffered saline containing 1.0% (w/v) Tween 80 and 0.002% (w/v) kanamycin sulfate, and has a diffusion area of 1.13 $cm^3$. The sample volume was 200 μl for each measurement, and the same amount of acceptor compartment

solution was replenished. The stirring speed was 600 rpm, the temperature was 32±0.5°C, and the number of repetitions per test was three times. Sampling was performed at 1, 3, 7, 13, 21, and 24 hours on day 1, and every 48 hours from days 2 to 10. Each sample collected was analyzed according to the antifungal agent content analysis method described above, and the results were confirmed as the average value of cumulative permeate concentration (ug/ml) and standard deviation. The results are shown in FIGS 1, 2, and 3.

FIG. 1 is a graph showing the cumulative permeate concentration results of luriconazole for 10 days according to Control Example 1, Comparative Example 1, and Manufacturing Examples 1 to 5. Referring to FIG. 1, Manufacturing Example 3 showed the highest permeate concentration compared to Control Example 1 and Comparative Example 1, with permeate concentrations of 6.5 times and 1.4 times on the first day, cumulative permeate concentrations of 5.3 times and 1.4 times on the second day, cumulative permeate concentrations of 4.7 times and 1.7 times on the sixth day, and cumulative permeate concentrations of 3.9 times and 1.8 times on the final day, the 10th day. Therefore, it can be seen that Manufacturing Example 3, which contains 30 wt% of crystal growth inhibitor (weight ratio of 1,2-pentanediol: acetyltributyl citrate is 1:2) and 10 wt% of keratin affinity inhibitor (cystamine dihydrochloride) based on the total weight of the composition, can treat nail tinea and skin tinea more effectively than Control Example 1, which does not contain crystal growth inhibitor and keratin affinity inhibitor, and Comparative Example 1, which does not contain keratin affinity inhibitor.

FIG. 2 is a graph showing the cumulative permeate concentration results of efinaconazole for 10 days according to Control Example 2, Comparative Example 2, and Manufacturing Examples 6 to 10. Referring to FIG. 2, Manufacturing Example 8 showed the highest permeate concentration of 6.1 times and 1.3 times in the permeate concentration on the first day, 4 times and 1.2 times in the cumulative permeate concentration on the second day, 3.5 times and 1.3 times in the cumulative permeate concentration on the sixth day, and 4.5 times and 1.5 times in the final day, the 10th day, compared to Control Example 2 and Comparative Example 2. Therefore, it can be seen that Manufacturing Example 8, which contains 30 wt% of crystal growth inhibitor (weight ratio of 1,2-pentanediol: acetyltributyl citrate is 1:2) and 10 wt% of keratin affinity inhibitor (cystamine dihydrochloride) based on the total weight of the composition, can treat nail tinea and skin tinea more effectively than Control Example 2, which does not contain crystal growth inhibitor and keratin affinity inhibitor, and Comparative Example 2, which does not contain keratin affinity inhibitor.

FIG. 3 is a graph showing the cumulative permeate concentration results of tavaborole for 10 days according to Control Example 3, Comparative Example 3, and Manufacturing Examples 11 to 15. Referring to FIG. 3, Manufacturing Example 13 showed the highest permeate concentration of 6.3 times and 1.2 times in the permeate concentration on the first day, 5.4 times and 1.2 times in the cumulative permeate concentration on the second day, 4.4 times and 1.5 times in the cumulative permeate concentration on the sixth day, and 5.0 times and 1.7 times in the final day, the 10th day, compared to Control Example 3 and Comparative Example 3. Therefore, it can be seen that Manufacturing Example 13, which contains 30 wt% of crystal growth inhibitor (weight ratio of 1,2-pentanediol: acetyltributyl citrate is 1:2) and 10 wt% of keratin affinity inhibitor (cystamine dihydrochloride) based on the total weight of the composition, can treat nail tinea and skin tinea more effectively than Control Example 3, which does not contain crystal growth inhibitor and keratin affinity inhibitor, and Comparative Example 3, which does not contain keratin affinity inhibitor.

Test Example 7. Storage stability evaluation

Control Examples 1 to 3, Comparative Examples 1 to 3, Manufacturing Examples 3, 8 and 13 were each placed in 20 ml transparent vials (10 ml each), sealed hermetically, and stored at 50°C for 4 weeks. The UV-visible absorption spectra of the samples were measured initially and after 4 weeks at 50°C. Measurements were performed at 400 nm and 600 nm using a Mega-800 (Scinco company, Korea) spectrophotometer, and the evaluation results are shown in Tables 11 and 12 below.

[Table 11]

| Absorbance at 400 nm | | | |
|---|---|---|---|
| Sample | | Initial | After 4 weeks |
| Luriconazole | Cont. Ex. 1 | 0.106±0.001 | 0.118±0.003 |
| | Comp. Ex. 1 | 0.111±0.003 | 0.116±0.002 |
| | Manuf. Ex. 3 | 0.121±0.003 | 0.123±0.001 |
| Efinaconazole | Cont. Ex. 2 | 0.118±0.004 | 0.134±0.005 |
| | Comp. Ex. 2 | 0.124±0.001 | 0.129±0.003 |
| | Manuf. Ex. 8 | 0.138±0.003 | 0.140±0.002 |

(continued)

| Absorbance at 400 nm | | | |
|---|---|---|---|
| Sample | | Initial | After 4 weeks |
| Tavaborole | Cont. Ex. 3 | 0.101±0.002 | 0.114±0.004 |
| | Comp. Ex. 3 | 0.105±0.002 | 0.112±0.003 |
| | Manuf. Ex. 13 | 0.112±0.003 | 0.114±0.001 |

[Table 12]

| Absorbance at 600 nm | | | |
|---|---|---|---|
| Sample | | Initial | After 4 weeks |
| Luriconazole | Cont. Ex. 1 | 0.054±0.002 | 0.060±0.004 |
| | Comp. Ex. 1 | 0.062±0.003 | 0.066±0.003 |
| | Manuf. Ex. 3 | 0.071±0.002 | 0.072±0.002 |
| Efinaconazole | Cont. Ex. 2 | 0.067±0.004 | 0.075±0.005 |
| | Comp. Ex. 2 | 0.075±0.001 | 0.079±0.003 |
| | Manuf. Ex. 8 | 0.086±0.002 | 0.088±0.001 |
| Tavaborole | Cont. Ex. 3 | 0.048±0.003 | 0.055±0.004 |
| | Comp. Ex. 3 | 0.056±0.002 | 0.060±0.002 |
| | Manuf. Ex. 13 | 0.063±0.002 | 0.064±0.001 |

As shown in Table 11 to Table 12, Manufacturing Examples 3, 8 and 13 obtained very stable results compared to Control Examples 1, 2 and 3 and Comparative Examples 1, 2 and 3. At 400 nm absorbance, Manufacturing Examples 3, 8, and 13 showed an increase in absorbance of 1.4 to 1.8%, while Control Examples 1, 2, and 3 showed an increase of 11.3 to 13.6%, and Comparative Examples 1, 2, and 3 showed an increase of 4.0 to 6.7%. At 600 nm absorbance, Manufacturing Examples 3, 8, and 13 showed an increase in absorbance of 1.4 to 2.3%, while Control Examples 1, 2, and 3 showed an increase of 11.1 to 14.6%, and Comparative Examples 1, 2, and 3 showed an increase of 5.3 to 7.1%. Looking at the stability results, Control Examples 1, 2, and 3, which did not contain crystal growth inhibitor and keratin affinity inhibitor, had the worst stability, and Comparative Examples 1, 2, and 3, which only contained crystal growth inhibitor, also had poor stability. In comparison, Manufacturing Examples 3, 8 and 13, which contained 30 wt% of crystal growth inhibitor (1,2-pentanediol: acetyltributyl citrate weight ratio of 1:2) and 10 wt% of keratin affinity inhibitor (cystamine dihydrochloride) based on the total weight of the composition, had the best stability.

As described above, according to various embodiments of the present invention, the crystal growth of the poorly soluble antimicrobial agents luriconazole, efinaconazole, and tavaborole, which are active ingredients, can be inhibited, thereby improving the solubility of the drugs. Accordingly, the phenomenon of crystal precipitation does not occur even when temperature changes during long-term storage, and when the drug is applied to the affected area of the skin or nail, the formation of fine precipitates in the affected area after the volatile solvent evaporates is prevented, thereby significantly improving the drug permeability.

And, according to various embodiments of the present invention, there is an advantage of preventing crystal precipitation by suppressing crystal growth phenomenon that may occur during the storage period of the drug and after drug application, and significantly improving the drug penetration effect and antimicrobial power by suppressing affinity with keratin of the skin and nail.

In addition, according to various embodiments of the present invention, the stability of the formulation is improved by inhibiting crystal growth of a poorly soluble antimicrobial agent, the antimicrobial activity is increased by preventing crystals from precipitating after evaporation of a volatile solvent when applied to the skin and nails, and the keratin affinity of the antimicrobial agent to the nails is inhibited to effectively increase drug permeability, thereby maximizing the treatment and prevention effect of tinea.

In addition, according to various embodiments of the present invention, when applying the drug to the affected area of the skin and nail, the keratin affinity of the antimicrobial agent, which is an active ingredient, is suppressed, thereby further increasing the drug permeability, so there is an excellent advantage in the prevention and treatment effect of tinea.

In addition, according to various embodiments of the present invention, the effect of preventing crystal

precipitation after drug application is excellent, so that the drug compliance is high, and the drug permeability and antimicrobial power in the skin and nails are very excellent.

In addition, it does not contain cyclomethicone and N-methylpyrrolidone, which are regulated substances contained in products currently on the market, and ethylenediamine tetraacetic acid salt, which has poor biodegradability, so it is less harmful and more environmentally friendly than products containing these ingredients, and can be widely used as a pharmaceutical composition for topical preparations for the prevention or treatment of tinea.

In other words, the pharmaceutical composition according to various embodiments of the present invention is composed of an environmentally friendly and non-hazardous composition because it does not use cyclomethicone, an environmentally hazardous substance, ethylenediamineacetic acid, an environmentally harmful substance, or methylpyrrolidone, a carcinogenic substance. Therefore, it has the advantage of excluding hazardous ingredients from currently commercial products, inhibiting crystal growth of antifungal agents to improve solubility, and reducing keratin affinity to significantly improve drug penetration ability and antimicrobial effects in the skin and nails.

In various embodiments of the present invention, cyclomethicone and N-methylpyrrolidone, which are hazardous substances, are not used as excipients, and ethylenediamine tetraacetic acid salt, which has poor biodegradability, is not used as a chelating agent, so there is an advantage of being non-hazardous and very environmentally friendly.

Finally, according to various embodiments of the present invention, the crystal growth inhibitor as an auxiliary component of the pharmaceutical composition inhibits the crystal growth of the drug, so that precipitation does not occur during long-term storage, and even after applying the drug to the affected area, crystals do not form, thereby improving the permeability of the drug, and the keratin affinity inhibitor as an auxiliary component can significantly reduce the affinity of the drug for keratin, thereby further improving the permeability of the drug. In other words, pharmaceutical compositions according to various embodiments significantly enhance drug penetration by simultaneously performing the dual functions of crystal growth inhibition and keratin affinity inhibition, and also exhibit a tendency to significantly increase antimicrobial activity. This enhanced drug penetration and antimicrobial activity can contribute to maximizing the preventive or therapeutic effects of skin and nail tinea.

In addition, the pharmaceutical composition according to various embodiments does not use cyclomethicone, an environmentally hazardous substance, ethylenediamineacetic acid salt, an environmentally harmful substance, and methylpyrrolidone, a carcinogenic substance, and is composed of an environmentally friendly and non-toxic composition, so it is excellent in safety and environmental friendliness for the human body.

As described above, the present invention has been specifically described by way of examples. However, since the above-described examples are merely preferred examples of the present invention, the present invention should not be understood as being limited to the above-described examples, and the scope of the present invention should be understood by the claims described below and their equivalents.

## Claims

**1.** A pharmaceutical composition for preventing or treating nail and skin tinea, comprising:

an antifungal agent;
a crystal growth inhibitor; and
a keratin affinity inhibitor.

**2.** The pharmaceutical composition for preventing or treating nail and skin tinea of claim 1, wherein the content of the antifungal agent is 0.1 to 20 wt% based on the total weight of the composition, and
the antifungal agent comprises at least one selected from luriconazole, luriconazole salt, efinaconazole, efinaconazole salt, tavaborole, and tavaborole salt.

**3.** The pharmaceutical composition for preventing or treating nail and skin tinea of claim 1, wherein the content of the crystal growth inhibitor is 0.1 to 70 wt% based on the total weight of the composition,

the crystal growth inhibitor comprises 1,2-pentanediol; and an ester compound,
the weight ratio of the 1,2-pentanediol and the ester compound is 1:2, and
the ester compound is at least one selected from the group consisting of acetyltributyl citrate, acetyltriethyl citrate, triethyl citrate, and diethyl carbonate.

**4.** The pharmaceutical composition for preventing or treating nail and skin tinea of claim 1, wherein the keratin affinity inhibitor contains cystamine dihydrochloride.

5. The pharmaceutical composition for preventing or treating nail and skin tinea of claim 4, wherein the keratin affinity inhibitor further comprises at least one selected from lauryl choline, tricaprylin, and octenidine dihydrochloride, and the content of the keratin affinity inhibitor is 0.01 to 30 wt% based on the total weight of the composition.

6. The pharmaceutical composition for preventing or treating nail and skin tinea of claim 1, wherein the pharmaceutical composition for preventing or treating nail and skin tinea further comprises a chelating agent,

the content of the chelating agent is 0.0001 to 5 wt% based on the total weight of the composition, and
the chelating agent comprises trisodium ethylenediamine disuccinate.

7. The pharmaceutical composition for preventing or treating nail and skin tinea of claim 1, wherein the additive comprises at least one selected from a vehicle, an antioxidant and a pH adjuster.

8. The pharmaceutical composition for preventing or treating nail and skin tinea of claim 1, wherein the pharmaceutical composition for preventing or treating nail and skin tinea has at least one formulation selected from a liquid, an aerosol, a stick, and a semi-solid preparation, wherein the semi-solid preparation includes at least one selected from gel, a lotion, a cream, and an ointment,

the pH of the pharmaceutical composition for preventing or treating nail and skin tinea is 2 to 10, and
the density of the pharmaceutical composition for preventing or treating nail and skin tinea is 0.01 to 2g/$cm^3$.

9. The pharmaceutical composition for preventing or treating nail and skin tinea of claim 1, wherein the pharmaceutical composition for preventing or treating nail and skin tinea does not contain cyclomethicone, methylpyrrolidone and ethylenediamine tetraacetic acid salt,
the pharmaceutical composition for preventing or treating nail and skin tinea has a moisture content of 0.1 to 90 wt% as measured by the Karl Fischer method, a surface tension of the pharmaceutical composition for preventing or treating nail and skin tinea is 1 to 76dyn/cm, and a daily application amount of the pharmaceutical composition for preventing or treating nail and skin tinea when applied topically is 0.001 to 60g/day.

【Figure 1】

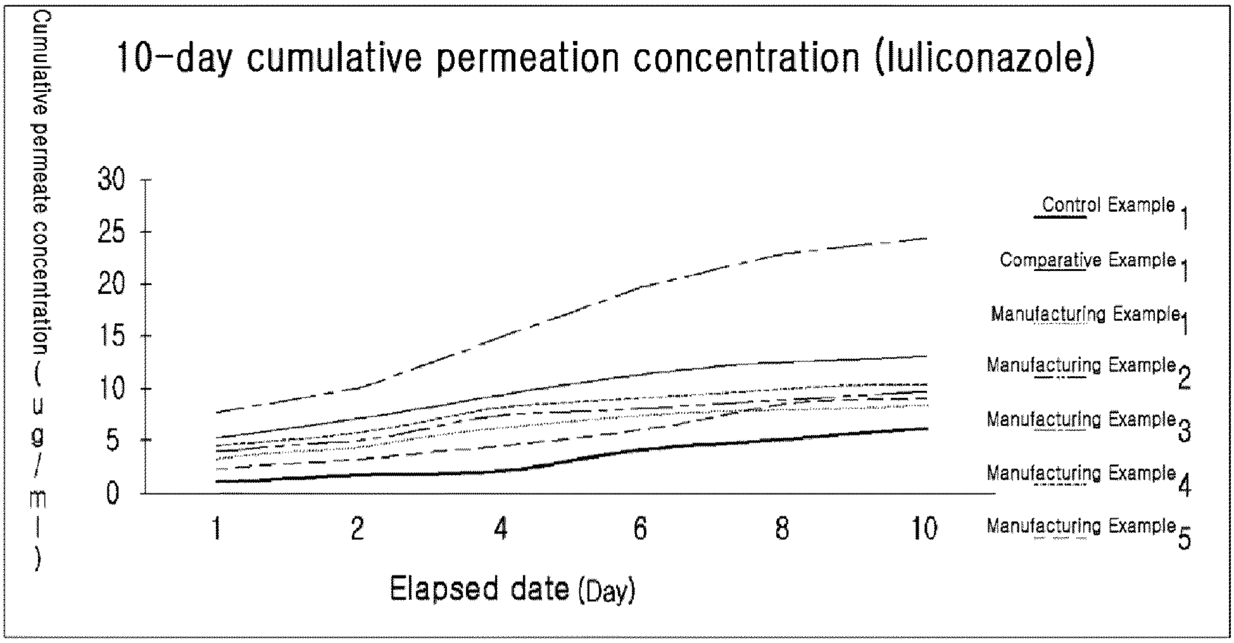
10-day cumulative permeation concentration (luliconazole)
Cumulative permeate concentration (ug/ml)
30
25
20
15
10
5
0
1
2
4
6
8
10
Elapsed date (Day)
Control Example 1
Comparative Example 1
Manufacturing Example 1
Manufacturing Example 2
Manufacturing Example 3
Manufacturing Example 4
Manufacturing Example 5

【Figure 2】

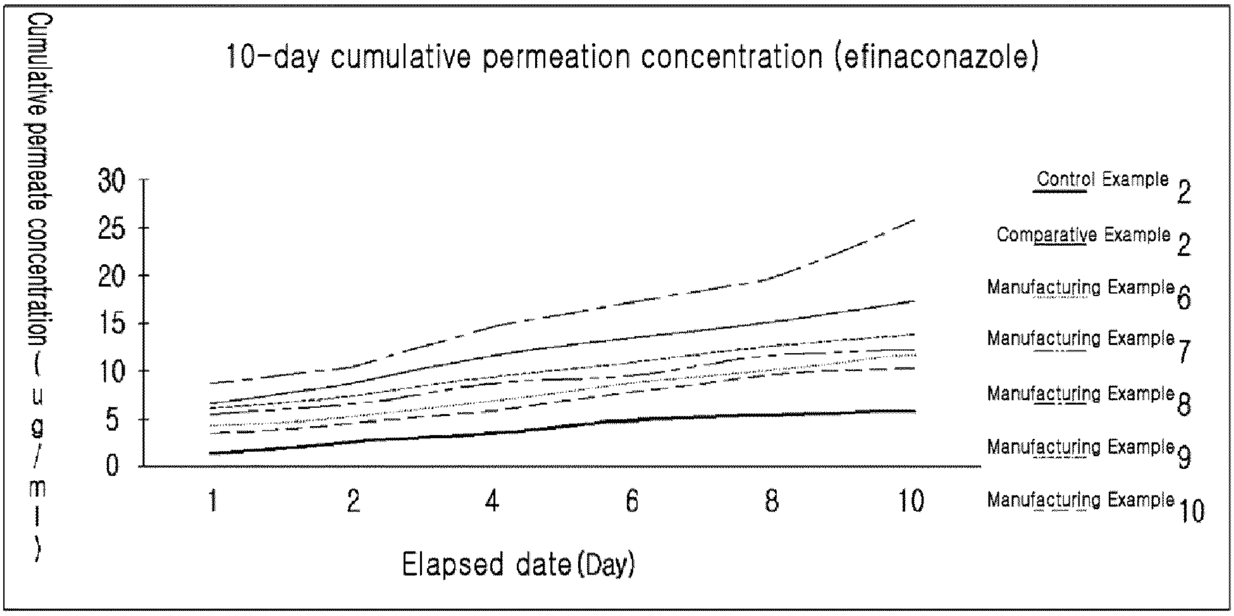


【Figure 3】

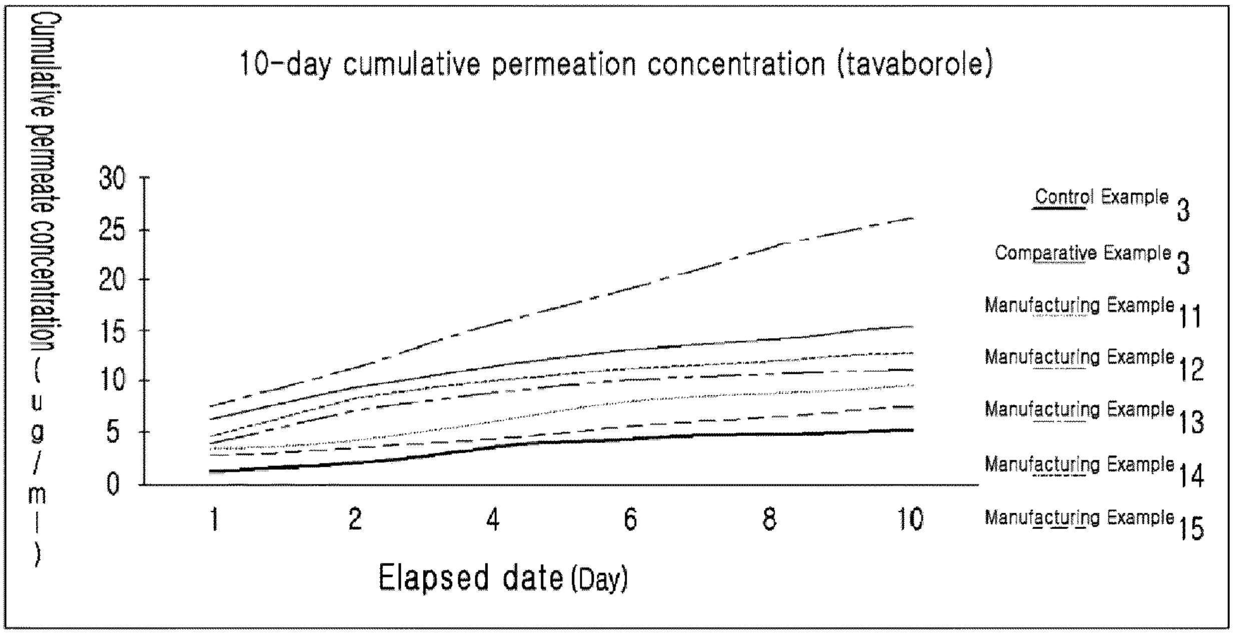

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BYUNG HO OH** ; **KYU JOONG AHAN**. Drug Therapy of Dermatophytosis. *J Korean Med. Assoc.*, 2009, vol. 52 (11), 1109-1114
- **UWE WOLLINA** ; **HOLGER HAENSSLE**. *Dtsch Arztebl Int.*, 2016, vol. 113 (29-30), 509-518